# EUROPEAN PATENT APPLICATION

(11) **EP 4 480 502 A1**
(43) Date of publication of application: **25.12.2024**
(21) Application number: 23756347.3
(22) Date of filing: 14.02.2023
(51) Int. Cl.: A61K 51/10, A61K 39/395, A61K 47/68, A61P 35/00

(54) **RADIOACTIVE COMPLEX OF ANTI-VEGF ANTIBODY, AND RADIOPHARMACEUTICAL**

(30) Priority: 15.02.2022 JP 2022021663
(71) Applicant: Nihon Medi-Physics Co., Ltd., Tokyo 136-0075 (JP)
(72) Inventor: NARITA, Yudai, Tokyo 136-0075 (JP); TAKENAKA, Fumiaki, Tokyo 136-0075 (JP); KISHIMOTO, Satoshi, Tokyo 136-0075 (JP); OTSUKA, Yuta, Tokyo 136-0075 (JP); HANADA, Takahisa, Tokyo 136-0075 (JP); ABE, Yuju, Tokyo 136-0075 (JP)
(74) Representative: J A Kemp LLP
(86) International application number: PCT/JP2023/004911
(87) International publication number: WO 2023/157822

(57) **Abstract**

the present invention aims to provide a novel radiolabeled anti-VEGF antibody applicable to nuclear medicine diagnosis or radiotherapy. The conjugate of the present invention is a conjugate of an antibody which is site-specifically modified with an antibody-modification peptide and a chelating agent, wherein the antibody is an anti-VEGF antibody and a metal radionuclide is chelated to the chelating agent.

## Description

### [Technical Field]

The present invention relates to a radioactive conjugate of an anti-VEGF antibody, and a radiopharmaceutical.

### [Background Art]

Vascular endothelial growth factor (VEGF) is a group of glycoproteins involved in vasculogenesis and angiogenesis. It binds as a ligand to the vascular endothelial growth factor receptor (VEGFR) on the surface of vascular endothelial cells, and has the functions of stimulating cell division, migration, and differentiation, and promoting vascular permeability of microvessels. In addition, it is known to be involved in the activation of monocytes and macrophages. There are seven growth factors of VEGF-A, VEGF-B, VEGF-C, VEGF-D, VEGF-E, PlGF (placental growth factor)-1, and PlGF-2, that are involved in vasculogenesis, angiogenesis, and lymphangiogenesis. These are collectively referred to as the "VEGF family". Furthermore, some VEGF family members have several subtypes due to alternative splicing. For example, in human, VEGF-A generally exist in four types, with 121 amino acids (VEGF-A₁₂₁), 165 amino acids (VEGF-A₁₆₅), 189 amino acids (VEGF-A₁₈₉), and 206 amino acids (VEGF-A₂₀₆), and rare subtypes such as VEGF-A₁₄₅ and VEGF-A₁₈₃ have also been reported. VEGF-B₁₆₇ and VEGF-B₁₈₆ are known for VEGF-B.

Anti-VEGF antibodies act, for example, by preventing VEGF from binding to cell receptors, by preventing activation of vascular endothelial cells after the binding of VEGF to cell receptors, or by killing cells activated by VEGF. Examples of the anti-VEGF antibody include bevacizumab (trade name: avastin), aflibercept beta (trade name: ZALTRAP), aflibercept (trade name: Eylea), and brolucizumab (trade name: Beovu).

Antibody drugs have high target selectivity and relatively few side effects; on the contrary, the efficacy thereof is insufficient in some cases. In contrast, chemotherapeutic agents have strong efficacy, but low target selectivity. Accordingly, the minimum effective dose required to kill cancer cells is high, whereas the maximum tolerated dose is low because the dose cannot be increased much in view of toxicity, and the therapeutic dose range is problematically narrow.

One technology that compensates for these shortcomings is antibody-drug conjugate (ADC). ADC uses antibodies to narrow down target cells (e.g. cancer cells) and delivers a drug (payload) attached to the antibody directly into the target cells. As a result, the biological activity of the drug is exerted target cell-specifically while avoiding an influence on normal cells.

Using ADC, it is possible to selectively deliver large amounts of chemotherapeutic agents to the target cells, and as a result, smaller doses show effects, the amount of chemotherapeutic agent that reaches normal cells decreases, the maximum tolerated dose increases, and the therapeutic dose range is expected to become wide.

One approach to ADC is a radioimmunoconjugate. In the radioimmunoconjugate, a radionuclide is used instead of payload.

As a site-specific chemical modification to add a function without affecting the specificity of antibody to a target molecule, a technique has been reported in which an amino acid capable of binding to a crosslinking agent is introduced into a peptide that specifically or selectively binds to an antibody (hereinafter also referred to as an antibody-modification peptide), and the amino acid is modified with a crosslinking agent to prepare an antibody-modification peptide that is site-specifically modified with a crosslinking agent, and then the antibody-modification peptide is used to site-specifically modify the antibody (Patent Literatures 1, 2).

In addition, Patent Literatures 3, 4 disclose techniques relating to some methods for labeling an antibody with a radionuclide.

In addition, Non Patent Literature 1 has reported the results of labeling an anti-VEGF antibody bevacizumab with zirconium-89, administering same to humans, and taking images by positron emission tomography (PET) method. In this example, a method is disclosed in which Fe-N-suc-Df-TFP is randomly bound to the amino groups of bevacizumab, and zirconium-89 is chelated to deferoxamine (Df).

### [Citation List]

### [Patent Literature]

[Patent Literature 1]
   WO 2017/217347
[Patent Literature 2]
   WO 2019/203191
[Patent Literature 3]
   WO 2019/125982
[Patent Literature 4]
   WO 2020/229974

### [Non Patent Literature]

[Non Patent Literature 1] EJNMMI Res. 2014 Dec; 4(1):35. doi: 10.1186/s13550-014-0035-5.

### [Summary of Invention]

Bevacizumab specifically binds to human VEGF, thereby inhibiting the binding of VEGF to VEGF receptor expressed on vascular endothelial cells, and inhibits the biological activity of VEGF, thereby suppressing angiogenesis in tumor tissues and inhibiting tumor growth. However, sufficient effects cannot be obtained when used alone, and combined use with other drugs (anticancer agents) has been essential in clinical practice.

The effect of radiotherapy using bevacizumab is not yet known.

Moreover, site-specifically radionuclide-modified bevacizumab is not yet known.

Therefore, the present invention aims to provide a novel radiolabeled anti-VEGF antibody applicable to nuclear medicine diagnosis or radiotherapy.

One embodiment of the present invention is a conjugate of an antibody site-specifically modified with an antibody-modification peptide and a chelating agent, wherein the antibody is an anti-VEGF antibody and a metal radionuclide is chelated to the chelating agent.

Another embodiment of the present invention is a radiopharmaceutical containing the above-mentioned conjugate as an active ingredient.

The "linking" in the present invention means both direct connection and indirect connection, unless otherwise specified.

According to the present invention, a novel radiolabeled anti-VEGF antibody applicable to nuclear medicine diagnosis or radiotherapy is provided.

### [Brief Description of Drawings]

[Fig. 1]

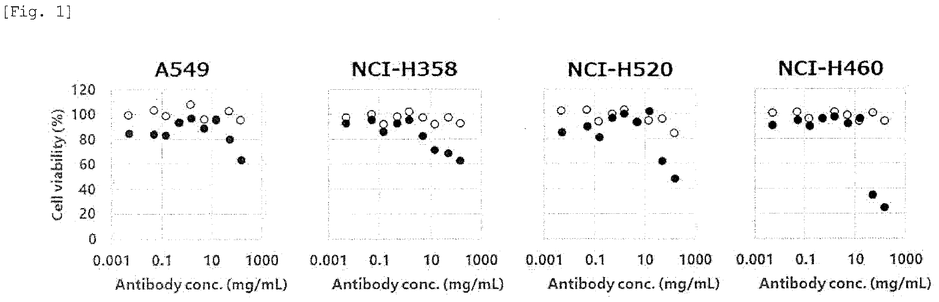

   Fig. 1 is a diagram showing the evaluation results of the in vitro efficacy (Cytotoxic effect) of one embodiment (²²⁵Ac-CCAP-bevacizumab1) of the radioactive conjugate of the present invention. In this Figure, the results with unlabeled bevacizumab are shown for comparison.
[Fig. 2]

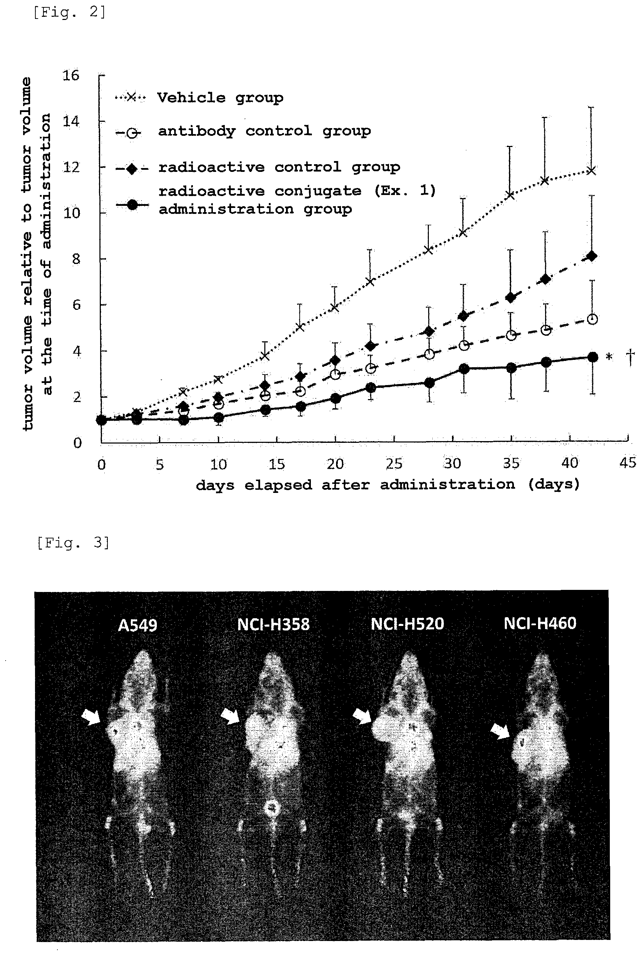

   Fig. 2 is a diagram showing the evaluation results of the in vivo efficacy (antitumor effect) of one embodiment (²²⁵Ac-CCAP-bevacizumab1) of the radioactive conjugate of the present invention.
[Fig. 3]
   Fig. 3 is a diagram showing a representative example of a PET image of one embodiment (⁸⁹Zr-CCAP-bevacizumab1) of the radioactive conjugate of the present invention in a tumor-bearing mouse.

### [Description of Embodiments]

### (1) Radioactive conjugate

The present invention is a conjugate of an antibody site-specifically modified with an antibody-modification peptide and a chelating agent, wherein the antibody is an anti-VEGF antibody and a metal radionuclide is chelated to the chelating agent (hereinafter also to be referred to as the radioactive conjugate of the present invention).

### (1-1) Metal radionuclide

The metal radionuclide contained in the radioactive conjugate of the present invention is a radionuclide that emits α-ray, a radionuclide that emits β-ray, a radionuclide that emits positron, or a radionuclide that emits γ-ray. When the radioactive conjugate of the present invention is used for cancer therapy, it is preferable to use a radionuclide that emits α-ray or a radionuclide that emits β-ray. When the radioactive conjugate of the present invention is used for cancer diagnosis or detection, it is preferable to use a radionuclide that emits positron, or a radionuclide that emits γ-ray. Examples of the radionuclide that emits α-ray include Bi-212, Bi-213, Ac-225, and Th-227. Examples of the radionuclide that emits β-ray include Cu-64, Y-90, and Lu-177. Examples of the radionuclide that emits positron include Cu-64, Ga-68, Y-86, and Zr-89. Examples of the radionuclide that emits γ-ray include Tc-99m and In-111. The metal radionuclide to be contained in the radioactive conjugate of the present invention is more preferably Ga-68, Zr-89, Y-90, In-111, Lu-177, or Ac-225.

### (1-2) Antibody

The antibody contained in the radioactive conjugate of the present invention is an immunoglobulin that specifically binds to the vascular endothelial cell growth factor (VEGF) (hereinafter also to be referred to as the antibody to be used in the present invention). The antibody to be used in the present invention may be a polyclonal antibody or a monoclonal antibody, preferably a monoclonal antibody. The origin of the antibody is not particularly limited, and examples include antibodies of non-human animals, non-human mammals, and humans, preferably antibodies of human, rat, mouse, and rabbit. When the antibody is derived from species other than human, it is preferably chimerized or humanized using well-known techniques. The antibody to be used in the present invention may be a chimera antibody, a humanized antibody, or a human antibody. The antibody to be used in the present invention may be a bispecific antibody. The antibody may be an isolated antibody or a purified antibody. The antibody may be, for example, IgG. The antibody may be, for example, IgG1, IgG2 (e.g., IgG2a and IgG2b), IgG3, or IgG4.

The antibody used in the radioactive conjugate of the present invention is not limited as long as it is an anti-VEGF antibody. For example, bevacizumab, aflibercept beta, aflibercept, and brolucizumab can be mentioned, and bevacizumab is more preferred.

Bevacizumab is a monoclonal antibody against VEGF. It has the actions of suppressing angiogenesis and suppressing the growth and metastasis of tumors by inhibiting the function of VEGF. It is one of the molecular targeted drugs, is used as an anticancer agent, and is expected to be a therapeutic drug for age-related macular degeneration and diabetic retinopathy. Bevacizumab is a glycoprotein consisting of two light chain molecules of 214 amino acid residues (C₁₀₃₄H₁₅₉₁N₂₇₃O₃₃₈S₆; molecular weight: 23446.71) and two heavy chain molecules of 453 amino acid residues (C₂₂₃₅H₃₄₁₃N₅₈₅O₆₇₈S₁₆; molecular weight: about 49838.57: including one lacking one lysine residue at the C-terminus) produced in Chinese hamster ovary cells by expression of cDNA encoding a humanized monoclonal antibody consisting of the complementarity determining region of a mouse anti-VEGF monoclonal antibody and the framework region and constant region derived from human IgG1.

Bevacizumab is also known as AVASTIN (registered trademark) and various biosimilar products (bevacizumab BS), and can be produced, for example, according to a previous report (Presta et al., Cancer Res. (1997) 57: 4593-4599). Specifically, it has the following structure.

amino acid sequences and disulfide bonds L chain

DIQMTQSPSS LSASVGDRVT ITCSASQDIS NYLNWYQQKP GKAPKVLIYF
TSSLHSGVPS RFSGSGSGTD FTLTISSLQP EDFATYYCQQ YSTVPWTFGQ
GTKVEIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC (SEQ ID NO:1)

H chain EVQLVESGGG LVQPGGSLRL SCAASGYTFT NYGMNWVRQA PGKGLEWVGW
INTYTGEPTY AADFKRRFTF SLDTSKSTAY LQMNSLRAED TAVYYCAKYP
HYYGSSHWYF DVWGQGTLVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKK VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGK (SEQ ID NO:2)

Here, intramolecular disulfide bonds are shown with a solid line.
H chain E1: partial pyroglutamic acid; H chain N303: sugar chain bond; H chain K453: partial processing L chain C214-H chain C226, H chain C232-H chain C232, H chain C235-H chain C235: disulfide bonds

The sugar chain structure is shown below. 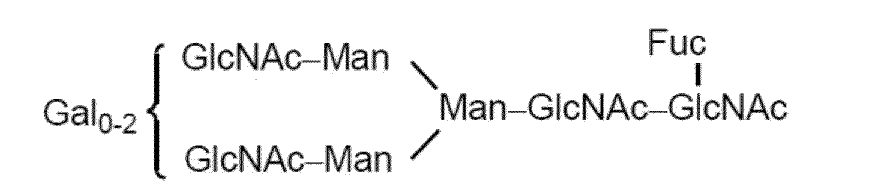 wherein Fuc is L-fucose, (Gal) ₀₋₂ is D-galactose with 0, 1, or 2 molecules, GlcNac is D-N-acetylglucosamine, and Man is D-mannose.

Aflibercept beta is a recombinant fusion glycoprotein, consisting of the second immunoglobulin (Ig)-like C2 domain of human vascular endothelial growth factor receptor (VEGFR) 1 (from the 1st to 104th), the third Ig-like C2 domain of human VEGFR2 (from the 105th to 205th), and the Fc domain of human IgG1 (from the 206th to 432nd). Aflibercept beta is produced in Chinese hamster ovary cells. Aflibercept beta is a glycoprotein (molecular weight: about 115,000) consisting of two subunits of 432 amino acid residues, and is commercially available under the trade name ZALTRAP (registered trademark).

Aflibercept is also available as Eylea (registered trade mark) and brolucizumab is available as Beovu (registered trade mark).

### (1-3) Chelating agent

In the present invention, the chelating agent is not particularly limited as long as it has a site in the structure thereof where metal radionuclide is coordinated. Examples of the chelating agent include CB-TE2A (1,4,8,11-tetraazabicyclo[6.6.2]hexadecane-4,11-diacetic acid), CDTA (cyclohexane-trans-1,2-diamine tetra-acetic acid), CDTPA (4-cyano-4-[[(dodecylthio)thioxomethyl]thio]pentanoic acid), DOTA (1,4,7,10-tetraazacyclododecane-1,4,7,10-tetraacetic acid), DOTMA ((1R,4R,7R,10R)-α,α',α",α'"-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-carboxyethyl)-1,4,7,10-tetraazacyclododecane-1,4,7,10-tetraacetic acid), DOTP (((1,4,7,10-tetraazacyclododecane-1,4,7,10-tetrayl)tetrakis(methylene))tetraphosphonic acid), DOTMP (1,4,7,10-tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (tetraazacyclododecane dimethanephosphonic acid), Deferoxamine (DFO), DTPA (Glycine, N,N-bis[2-[bis(carboxymethyl)amino]ethyl]-), DTPA-BMA (5,8-bis(carboxymethyl)-11-[2-(methylamino)-2-oxoethyl]-3-oxo-2,5,8,11-tetraazatridecan-13-oic acid), EDTA (2,2',2",2' ''-(ethane-1,2-diylbis(azanetriyl))tetraacetic acid), NOTA (1,4,7-triazacyclononane-1,4,7-triacetic acid), NOTP (1,4,7-triazacyclononane-1,4,7-triyltris(methylenephosphonic acid)), TETPA (1,4,8,11-tetraazacyclotetradecane-1,4,8,11-tetrapropionic acid), TETA (1,4,8,11-tetraazacyclotetradecane-N,N',N",N' "-tetraacetic acid), TTHA (3,6,9,12-tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N' ''-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N'",N""-pentaacetic acid), H4octapa (N,N'-bis(6-carboxy-2-pyridylmethyl)-ethylenediamine-N,N'-diacetic acid), H2bispa2 (6,6'-({9-hydroxy-1,5-bis (methoxycarbonyl)-2,4-di (pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid), H6phospa (N,N'-(methylenephosphonate)-N,N'-[6-(methoxycarbonyl)pyridin-2-yl]-methyl-1,2-diaminoethane), HP-D03A (hydroxypropyltetraazacyclododecanetriacetic acid), and porphyrin. A compound represented by the following formula (A) is preferred.

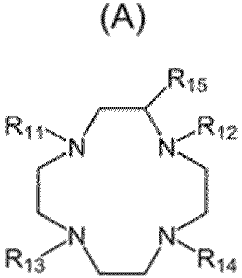

In the formula (A), R₁₁, R₁₂, R₁₃ and R₁₄ are each independently a group consisting of - (CH₂)ₚCOOH, - (CH₂)ₚC₅H₄N, -(CH₂)ₚPO₃H₂, - (CH₂)ₚCONH₂ or -(CHCOOH) (CH₂)ₚCOOH, R₁₅ is a hydrogen atom, and p is an integer of not less than 0 and not more than 3.

The compound represented by the formula (A) is preferably a compound containing a structure derived from 1,4,7,10-tetraazacyclododecane-1,4,7,10-tetraacetic acid (DOTA), or a derivative thereof. Specifically, the compound can contain, in its structure, a structure derived from one chelating agent selected from DOTA (1,4,7,10-tetraazacyclododecane-1,4,7,10-tetraacetic acid), DOTMA ((1R,4R,7R,10R)-α,α',α",α' "-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetrapropionic acid), L^{py} (1,4,7,10-tetrakis(pyridin-2-ylmethyl)-1,4,7,10-tetraazacyclododecane), DOTA-GA (α-(2-carboxyethyl)-1,4,7,10-tetraazacyclododecane-1,4,7,10-tetraacetic acid), DOTP (((1,4,7,10-tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), and D02P (tetraazacyclododecane dimethanephosphonic acid). More preferably, compounds represented by the following formulas (A-1) to (A-6) can be mentioned. The chelating agent to be used in the radioactive conjugate of the present invention is further preferably DOTA-GA (compound represented by the formula (A-6)). When the chelating agent used is DOTA-GA, the aforementioned chelating agent may be a stereoisomer (S-form, R-form) or a racemate, and in the racemate, the S-form and R-form stereoisomers may be mixed at any ratio.

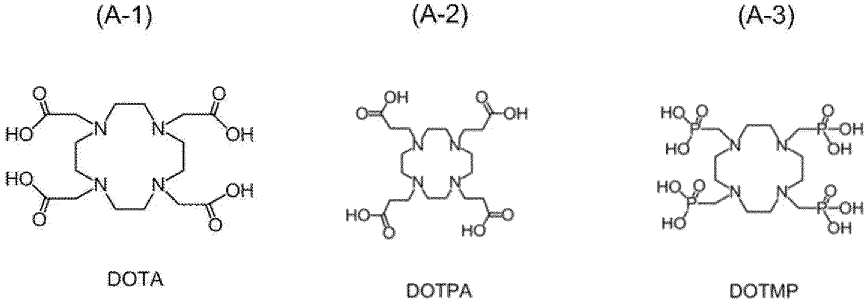

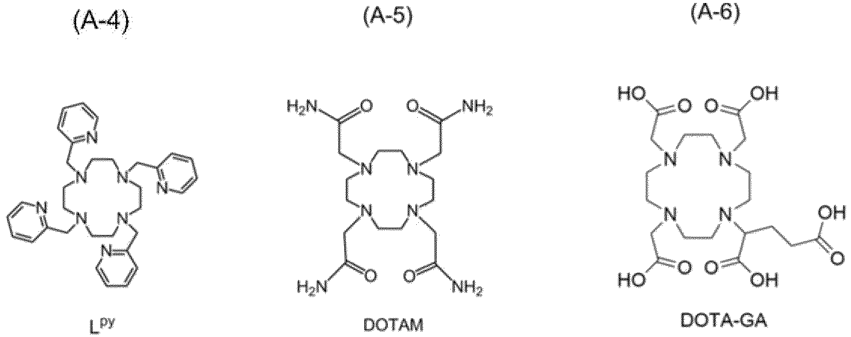

The chelating agent used in the present invention is preferably linked to the peptide via a linker (L). Specifically, in the radioactive conjugate of the present invention, the chelating agent and the linker (L) are preferably connected by a covalent bond. Therefore, in the radioactive conjugate of the present invention, some groups in the compound of the aforementioned chelating agent may be substituted by groups that form covalent bonds with the linker (L). For example, when the chelating agent used in the present invention is a compound represented by the formula (A), R₁₂ or R₁₅ may be substituted by a group that forms a covalent bond with the linker (L). Preferably, when R₁₂ is substituted by a group that forms a covalent bond with the linker (L), R₁₅ is a hydrogen atom, when R₁₂ is a group consisting of - (CH₂)ₚCOOH, - (CH₂)ₚC₅H₄N, - (CH₂)ₚPO₃H₂, - (CH₂)ₚCONH₂, or - (CHCOOH) (CH₂)ₚCOOH, R₁₅ is substituted by a group that forms a covalent bond with the linker (L).

The covalent bond between the chelating agent and the linker (L) preferably does not include a thiourea bond. In this case, a complex with improved stability can be provided without impairing the medicinal effect. Examples of the covalent bond between the chelating agent and the linker (L) include a carbon-carbon bond, an amide bond, an ether bond, and an ester bond.

The connection between the chelating agent and the linker (L) can be formed, for example, by the reaction of an N-hydroxysuccinimide ester (NHS) group of the following formula (A-7) or (A-8), or a 2,6-dioxotetrahydro-2H-pyranyl group of the following formula (A-9), with the primary amine of linker (L) .

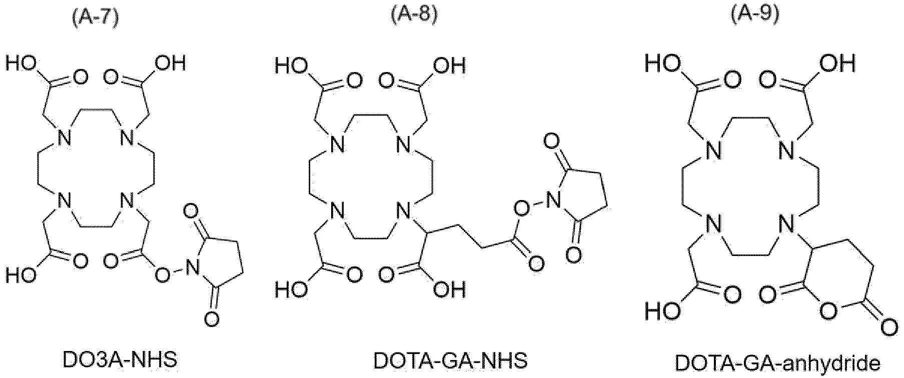

### (1-4) Antibody-modification peptide

The antibody-modification peptide used in the present invention is not particularly limited as long as it site-specifically modifies an antibody, preferably site-specifically modifies the Fc region, more preferably site-specifically modifies the lysine residue in the Fc region of an antibody. The activity of the antibody itself (antigen recognition action, neutralizing action, complement activating action and/or opsonin action) can be maintained thereby.

The antibody-modification peptide used in the present invention may be a linear peptide or a cyclic peptide, and a cyclic peptide is preferred. More preferably, it contains a peptide consisting of not less than 13 and not more than 17 amino acid residues represented by the following formula (i) and is modified with a crosslinking agent. In the explanation of formula (i), the left side of the paper surface of the amino acid sequence indicates the N-terminal side, and the right side of the paper surface of the amino acid sequence indicates the C-terminal side.

### (Xa) -Xaa₁-(Xb)-Xaa₂-(Xc)-Xaa₃-(Xd) ·· (i)

In the formula (i), Xa, Xb, Xc and Xd are each continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa₁ and Xaa₃ are each independently
an amino acid residue derived from an amino acid having a thiol group in the side chain or an amino acid residue derived from an amino acid having a haloacetyl group in the side chain, provided that either Xaa₁ or Xaa₃ is an amino acid residue derived from an amino acid having a thiol group, and preferably Xaa₁ and Xaa₃ are linked to form a ring structure, and
Xaa₂ is a lysine residue, arginine residue, cysteine residue, aspartic acid residue, glutamic acid residue, 2-aminosuberic acid, or diaminopropionic acid, preferably a lysine residue, and modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and anti-VEGF antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

At least one of Xaa₁ and Xaa₃ is an amino acid residue derived from an amino acid having a thiol group in the side chain, and the amino acid may be the same or different. Examples of the amino acid having a thiol group in the side chain include cysteine and homocysteine. Such amino acid residues are preferably bonded by a disulfide bond, or a sulfide group is preferably bonded thereto via a structure shown by the following formula (4). In the formula (4), the broken line indicates the binding part with the sulfide group.

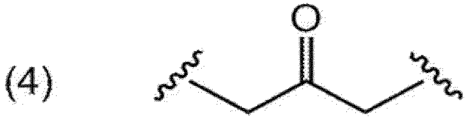

Instead of the aforementioned combination of Xaa₁ and Xaa₃, one of Xaa₁ and Xaa₃ may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Preferably, the antibody-modification peptide used in the present invention has an amino acid sequence consisting of 13 to 17 amino acid residues represented by the following formula (i)': (X₁₋₃)-C-(Xaa₃' ) -(Xaa₄') -H-(Xaa₁') -G-(Xaa₂')-L-V-W-C-(X₁₋₃) (i) '

In the formula (i)', each X is independently any amino acid residue other than cysteine,
C is a cysteine residue,
H is a histidine residue,
Xaa₁' is a lysine residue, a cysteine residue, an aspartic acid residue, a glutamic acid residue, 2-aminosuberic acid, or diaminopropionic acid,
G is a glycine residue,
Xaa₂' is a glutamic acid residue or an asparagine residue,
L is a leucine residue,
V is a valine residue,
W is a tryptophan residue,
Xaa₃' is an alanine residue, a serine residue or a threonine residue, and
Xaa₄' is a tyrosine residue or a tryptophan residue.

In the above-mentioned formula (i)', the notation X₁₋₃ at the N-terminus or C-terminus means that 1 to 3 consecutive amino acid residues X, which are independently any amino acid residue other than cysteine (C or Cys), are present, the amino acid residues that constitute the same may be the same or different residues, and preferably all three are not the same residue.

Among these, the amino acid sequence of the antibody-modification peptide preferably has any one of the following sequences (1) to (14), more preferably the following sequence (1), (2), (13) or (14). In the following amino acid sequences (1) to (14), (Xaa₂) is a lysine residue, a cysteine residue, an aspartic acid residue, a glutamic acid residue, a 2-aminosuberic acid, or a diaminopropionic acid, preferably a lysine residue, and is preferably modified with a crosslinking agent, and (Xaa₁) and (Xaa₃) are each a homocysteine residue. In the following amino acid sequences (1) to (14), the amino acids other than (Xaa₁), (Xaa₂) and (Xaa₃) are indicated by one-letter abbreviations.

(1) DCAYH(Xaa₂)GELVWCT (SEQ ID NO: 3)
(2) GPDCAYH(Xaa₂)GELVWCTFH (SEQ ID NO: 4)
(3) RCAYH(Xaa₂)GELVWCS (SEQ ID NO: 5)
(4) GPRCAYH(Xaa₂)GELVWCSFH (SEQ ID NO: 6)
(5) SPDCAYH(Xaa₂)GELVWCTFH (SEQ ID NO: 7)
(6) GDDCAYH(Xaa₂)GELVWCTFH (SEQ ID NO: 8)
(7) GPSCAYH(Xaa₂)GELVWCTFH (SEQ ID NO: 9)
(8) GPDCAYH(Xaa₂)GELVWCSFH (SEQ ID NO: 10)
(9) GPDCAYH(Xaa₂)GELVWCTHH (SEQ ID NO: 11)
(10) GPDCAYH(Xaa₂)GELVWCTFY (SEQ ID NO: 12)
(11) SPDCAYH(Xaa₂)GELVWCTFY (SEQ ID NO: 13)
(12) SDDCAYH(Xaa₂)GELVWCTFY (SEQ ID NO: 14)
(13) RGNCAYH(Xaa₂)GQLVWCTYH (SEQ ID NO: 15)
(14) G(Xaa₁)DCAYH(Xaa₂)GELVWCT(Xaa₃)H (SEQ ID NO: 16)

The peptides represented by the above-mentioned formula (i) or (i)' or the peptides having sequences (1) to (14) preferably have a linker (L) introduced at the N-terminus, and the C-terminus is amidated. Furthermore, Xaa₂ (or a portion corresponding to Xaa₂) of these peptides is modified with a crosslinking agent, whereby the peptides are covalently bound to the Fc region of an anti-VEGF antibody via the crosslinking agent.

The crosslinking agent can be appropriately selected by those of ordinary skill in the art, and can be a compound having at least two sites bindable to desired amino acid (e.g., lysine, cysteine, aspartic acid, glutamic acid, 2-aminosuberic acid, diaminopropionic acid, arginine, etc.). Examples thereof include, but are not limited to, a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride), and DSP (dithiobis(succinimidyl propionate)), and SBAP (succinimidyl 3-(bromoacetamido)propionate). Since crosslinking agents containing a succinimidyl group such as DSS or DSG react with primary amine present at the N-terminus, only the amino group of Xaa₂ can be specifically modified with DSS or DSG by blocking the N-terminus and then reacting the peptide with DSS or DSG. For example, a linker (L) may be introduced in advance into the N-terminus of the antibody-modification peptide, and then the reaction with DSS or DSG is performed. The succinimidyl group of DSS or DSG reacts with the Lys252 or Lys254 residue, preferably the Lys254 residue, according to the Eu numbering in an anti-VEGF antibody (e.g., bevacizumab), whereby the anti-VEGF antibody is site-specifically modified with the peptide. These Lys residues are present in the Fc region of human IgG, and even in the case of an anti-VEGF antibody other than bevacizumab, a person skilled in the art can align the amino acid sequence of the antibody and identify the corresponding Lys residue.

### (1-5) Linker (L)

Linker (L) is not particularly limited as long as it can link a chelating agent and an antibody-modification peptide in the radioactive conjugate of the present invention. The linker (L) used in the present invention is not particularly limited as long as it does not contain a thiourea bond and examples thereof include substituted or unsubstituted alkyl group, substituted or unsubstituted heteroalkyl group, polyethylene glycol (PEG) group, peptide, sugar chain, disulfide group, amide group, combination of these and the like.

In the present specification, linker (L) is a general term for linkers used for the connection of an anti-VEGF antibody modified with an antibody-modification peptide and a chelating agent, and includes antibody-modification linker (L₁) and chelate linker (L₂). The antibody-modification linker (L₁), which is described in detail later, is to be introduced into the N-terminal side of the antibody-modification peptide described in (1-4), and the chelate linker (L₂), which is described in detail later, is to be introduced into the functional group of the chelating agent described in (1-3).

The linker (L) used in the present invention may contain a binding site formed by a click reaction, and preferably, the antibody-modification linker (L₁) and the chelate linker (L₂) are bound by a click reaction. In the present invention, it is preferred that a thiourea bond is not present between the binding site formed by the click reaction and the chelating agent. In other words, it is preferred that the chelate linker (L₂) does not contain a thiourea bond. Here, the binding site formed by a click reaction is preferably a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

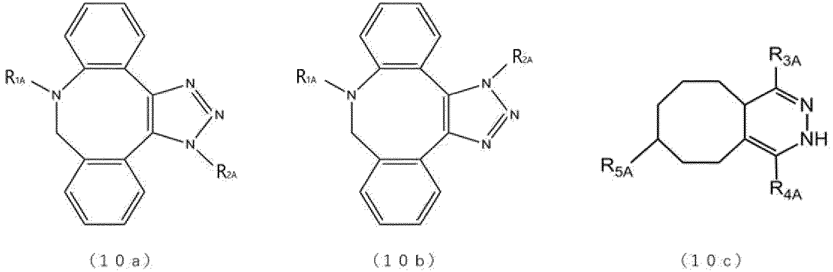

In the formula (10a) and the formula (10b), R_{1A} is a linkage site with a chelating agent, and R_{2A} is a linkage site with an antibody-modification peptide. In the formula (10c), one of R_{3A} and R_{4A} is a hydrogen atom, a methyl group, a phenyl group or a pyridyl group, and the other is a binding site with a chelating agent, and R_{5A} is a binding site with an antibody-modification peptide. In the formula (10a), the formula (10b) and the formula (10c), the linkage site with the antibody-modification peptide is linked with the peptide via the antibody-modification linker (L₁) , and the linkage site with the chelating agent is linked with the chelating agent via the chelate linker (L₂).

In the radioactive conjugate of the present invention, the antibody is site-specifically modified with an antibody-modification peptide, and the peptide and a chelating agent are linked via a linker (L). Thus, one molecule or two molecules of the chelating agent are conjugated to one molecule of the anti-VEGF antibody.

### (1-6) Production method of conjugate

The production method of the radioactive conjugate of the present invention includes two steps which are a conjugation step of conjugating a chelating agent and an anti-VEGF antibody, and a complex formation step of forming a complex of a metal radionuclide and a chelating agent. The conjugation step may be performed before the complex formation step or after the complex formation step.

In the conjugation step, the Fc region of an antibody is site-specifically modified with a chelating agent or linker (L) having the antibody-modification peptide represented by the aforementioned formula (i).

In the complex formation step, the chelating agent is chelated with a metal radionuclide (complex formation). The metal radionuclide used here is preferably used in a manner permitting ionization, more preferably in the form of an ion, from the viewpoint of increasing the complex formation efficiency. In the complex formation step, the order of addition of the metal radionuclide to the chelating agent does not matter as long as a complex can be formed with the metal radionuclide. For example, a solution in which radioactive metal ion is dissolved in a solvent mainly composed of water can be used as a radionuclide.

After complex formation, the obtained complex may be purified using a filtration filter, a membrane filter, a column filled with various fillers, chromatography or the like.

In the production method of the radioactive conjugate of the present invention, a conjugation step is preferably performed after the complex formation step.

In a more preferred embodiment, in complex formation step (A), a complex is formed between a metal radionuclide and a chelating agent having a first atomic group capable of click reaction as a substituent for enabling conjugation with the antibody. Then, in conjugation step (B), using an antibody-modification peptide shown by the aforementioned formula (i) and an antibody-modification linker (L₁) having a second atomic group capable of click reaction, a click reaction is performed between the peptide-modified antibody in which Fc region is site-specifically modified and the chelating agent with a formed complex which is obtained in step (A) to obtain the radioactive conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker (L₂) is preferably alkyne and the antibody-modification linker (L₁) is preferably azide, or the chelate linker (L₂) is preferably 1,2,4,5-tetrazine and the antibody-modification linker (L₁) is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a).

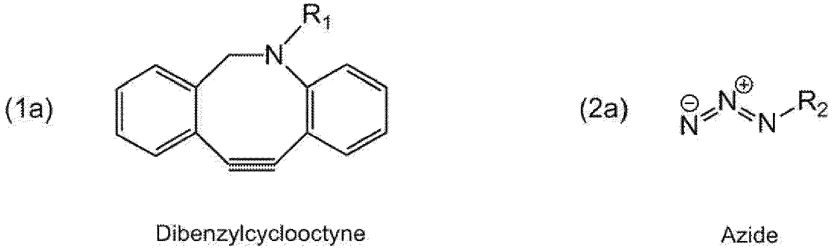

In the formula (1a), R₁ is a linkage site with a chelating agent, and in the formula (2a), R₂ is a linkage site with an antibody-modification peptide.

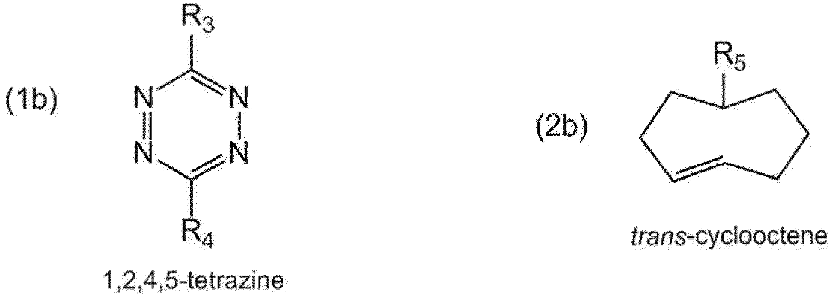

In the formula (1b), one of R₃ and R₄ is a linkage site with a chelating agent or an antibody-modification peptide, and the other is a hydrogen atom, a methyl group, a phenyl group or a pyridyl group. When the atomic group of the formula (1b) is linked with a chelating agent, R₅ is the linkage site with the antibody-modification peptide, and when the atomic group of the formula (1b) is linked with an antibody-modification peptide, R₅ is the linkage site with the chelating agent.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In the complex formation step (A), it is more preferred to use a compound having a structure represented by the following formula (ii):

A-B-C ··· (ii)

In the formula (ii), A is the aforementioned chelating agent, and the generic term for B and C is a chelate linker (L₂) .

In the formula (ii), B is represented by the following formula (iib):

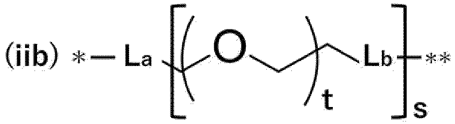

In the formula (iib), La and Lb are each independently a bond linker containing at least an amide bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

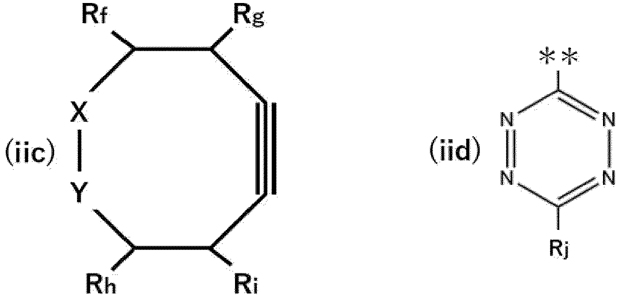

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (A) wherein R₁₁ to R₁₄ are -(CH₂)ₚCOOH, p is 1, R₁₅ is a binding site with B; or DO3A derivative or DOTAGA derivative wherein R₁₁ to R₁₄ are - (CH₂)ₚCOOH, p is 1, R₁₂ is a binding site (*) with B, and R₁₅ is a hydrogen atom is more preferred.

In the formula (ii), a DO3A-PEGt-DBCO wherein A is the above-mentioned DO3A, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred.

In the formula (ii), a DOTAGA-PEGt-DBCO derivative wherein A is the above-mentioned DOTAGA derivative, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred. It is more preferably the following DOTAGA-DBCO.

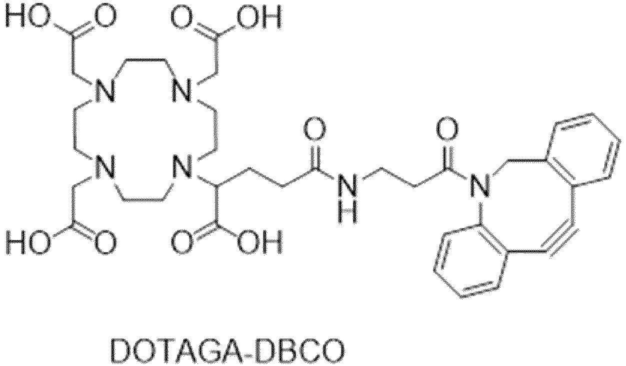

In the molar ratio of the chelating agent and metal radionuclide, as chelate site/metal radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 µmol/L, more preferably not less than 0.01 µmol/L, further preferably not less than 0.1 µmol/L, more preferably not less than 1 µmol/L, and the upper limit is preferably not more than 1000 µmol/L, more preferably not more than 100 µmol/L, further preferably not more than 10 µmol/L. For example, it is within the range of not less than 1 µmol/L and not more than 100 µmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

The antibody to be used in step (B) is a peptide-modified antibody in which Fc region (constant region) of anti-VEGF antibody as described in detail in the above-mentioned "(1-2) Antibody" is site-specifically modified using the antibody-modification peptide shown in the aforementioned formula (i), and an antibody-modification linker (L₂) having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker (L₂) may be one in which an antibody-modification peptide and a linker (L₂) represented by the following formula (S1) are bonded.

*-((Lᵢ)ₘ-Z)ₖ-Lᵢᵢ-AG2 (S1)

wherein * is a binding site with the N-terminal or C-terminal of peptide,
Lᵢ is a linker moiety of polyethylene glycol (PEG),
m is an integer of not less than 1 and not more than 50,
Z is a second linker moiety that binds (Lᵢ)ₘ and Lᵢᵢ,
k is 0 or 1,
Lᵢᵢ is the second PEG linker moiety, and
AG2 is a second atomic group.

In the aforementioned formula (S1), the structure of Z is not particularly limited as long as it is a linker structure that binds (Lᵢ)ₘ and Lᵢᵢ to each other, and includes, for example, an amino acid sequence consisting of not less than 1 and not more than 5 amino acid residues. In this case, the amino acid sequence contained in Z preferably contains a cysteine residue, and is more preferably bonded to L₂ via a thioether group formed by the bond between the thiol group of the cysteine residue and a maleimide group.

In the present invention, the polyethylene glycol (PEG) linker moiety constituting Lᵢᵢ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

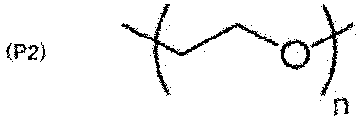

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker (L₂), an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an anti-VEGF antibody to obtain a peptide-modified antibody can be performed, for example, by dispersing the aforementioned antibody-modification peptide, an anti-VEGF antibody, a crosslinking agent, and a catalyst as necessary in an appropriate buffer, according to the description of WO 2017/217347. As the crosslinking agent, those mentioned above can be used. In addition, when binding the antibody-modification peptide and the anti-VEGF antibody, where necessary, buffer replacement including treating the solution containing the anti-VEGF antibody with an ultrafiltration filter, etc., and dispersing same in a buffer may be performed once or twice before binding with the antibody-modification peptide.

In one embodiment, the present disclosure relates to a method for producing a conjugate of an antibody-modification peptide and an anti-VEGF antibody, including a step of mixing an antibody-modification peptide modified with a crosslinking agent and an anti-VEGF antibody. By this step, a crosslinking reaction can occur between the antibody-modification peptide modified with a crosslinking agent and an anti-VEGF antibody (e.g., bevacizumab). The crosslinking reaction can occur site-specifically between the above-mentioned amino acid residue Xaa₂ of the antibody-modification peptide and the Lys252 or Lys254 residue, preferably the Lys254 residue, according to Eu numbering in human IgG Fc.

The conditions of the mixing step are not particularly limited as long as a crosslinking reaction occurs between the antibody-modification peptide and the anti-VEGF antibody. For example, the reaction can be performed by mixing the antibody-modification peptide and the anti-VEGF antibody in an appropriate buffer at room temperature (e.g., about 15°C to 30°C). The mixing step may be performed by adding as necessary an appropriate amount of a catalyst that promotes the crosslinking reaction.

In one embodiment, a solvent containing at least water is added to dissolve the anti-VEGF antibody. The solvent other than water includes, for example, dimethyl sulfoxide, acetonitrile, saline, and buffers such as sodium acetate buffer, ammonium acetate buffer, phosphate buffer, phosphate buffered saline, Tris buffer, HEPES buffer, tetramethylammonium acetate buffer, histidine buffer, and the like. When a buffer is used, the pH at 25°C is preferably set to 4.0 or more and 10.0 or less, more preferably 5.5 or more and 8.5 or less, from the aspect of the stability of the antibody. At the start of the crosslinking reaction, the concentration of the antibody is preferably set to 1.0 µmol/L or more as the lower limit and 1000 umol/L or less, more preferably 500 µmol/L or less, as the upper limit.

Then, an antibody-modification peptide modified with a crosslinking agent and, where necessary, a catalyst are added and the mixture is dispersed at 10°C or higher and 30°C or lower.

The mixing ratio of the antibody-modification peptide and the anti-VEGF antibody in the mixing step is not particularly limited. The molar ratio of the antibody-modification peptide and the anti-VEGF antibody can be set to, for example, 1:1 to 20:1, preferably 2:1 to 20:1 or 5:1 to 10:1.

In a preferred embodiment, antibody-modification peptide can be mixed with the anti-VEGF antibody at 0.5 to 2.2, preferably 0.8 to 1.8, (molar ratio) in the above-mentioned mixing step. In this case, an antibody in which one molecule of the antibody-modification peptide is bound to one anti-VEGF antibody molecule (hereinafter referred to as a "monovalent antibody") can be efficiently obtained.

The mixing time (reaction time) in the mixing step is not particularly limited as long as a crosslinking reaction occurs between the antibody-modification peptide and the anti-VEGF antibody. It is, for example, 1 min to 5 hr, preferably 10 min to 2 hr.

The peptide-modified antibody obtained through the above steps is a mixture containing an antibody in which one molecule of antibody-modification peptide is bound to one molecule of anti-VEGF antibody (i.e., "monovalent antibody") and an antibody in which two molecules of antibody-modification peptide are bound to one molecule of anti-VEGF antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is possible to use a column filled with various fillers. For example, a column filled with a filler in which a protein such as protein A, protein G, or the aforementioned antibody-modification peptide is bound to a carrier can be used. The shape of the carrier of the filler filled in such a column includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

The IgG-BP column is a column immobilized with an IgG-binding peptide. Divalent antibody cannot bind to the column because the binding sites are already occupied by the IgG-binding peptide, and only monovalent antibodies show affinity to the column. Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. In one preferred embodiment, the molar ratio of unmodified antibody to monovalent antibody in the first antibody composition is 4-47:53-96, preferably 4-30:70-96, more preferably 4-20:80-96, further preferably 4-10:90-96.

In this way, the first antibody composition or the second antibody composition separated and purified may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, at the start of step (B), the lower limit is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferable, and the range of not less than 0.1 mL and not more than 10 mL is more preferable.

As the concentrations of the chelating agent and the antibody in the reaction mixture, each independently, at the start of step (B), the lower limit is preferably not less than 0.001 µmol/L, more preferably not less than 0.01 µmol/L, further preferably not less than 0.1 µmol/L, further more preferably not less than 1.0 µmol/L, and the upper limit is preferably not more than 1000 µmol/L, more preferably not more than 100 µmol/L. For example, the range of not less than 0.1 µmol/L and not more than 1000 µmol/L is preferable, and the range of not less than 1 µmol/L and not more than 100 µmol/L is more preferable, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferable, and the range of not less than 10 min and not more than 20 hr is more preferable.

The obtained conjugate may be used as it is or purified using a filtration filter, a membrane filter, a column filled with various fillers, chromatography or the like.

In the conjugate produced by steps (A) and (B), the lysine residue in the Fc region of anti-VEGF antibody is specifically modified with a chelating agent. This conjugate comprises one or two molecules of the aforementioned chelating agent per one molecule of the antibody. The chelating agent site-specifically modifies the Fc region of the antibody of the present invention via a linker (L). The linker (L) is constituted of a chelate linker (L₂) that connects to a chelating agent, a first atomic group that connects to the linker (L₂), a second atomic group that can perform click reaction with the first atomic group, and an antibody-modification linker (L₁) that connects to the second atomic group (including an antibody-modification peptide represented by the above-mentioned formula (i)). Therefore, the linker (L) has a chemical structure derived from the first atomic group and the second atomic group. As such chemical structure, a triazole skeleton-containing structure represented by the aforementioned formula (10a) or (10b) or a pyridazine skeleton-containing structure represented by the aforementioned formula (10c) can be considered. Since the formula (10a) and the formula (10b) are isomers, they may be contained at any ratio.

### (1-7) Radiopharmaceutical

A radiopharmaceutical refers to a composition that contains the radioactive conjugate of the present invention and is in a form suitable for in vivo administration to a subject. The radiopharmaceutical may be, for example, a radioactive conjugate produced by the method shown in the aforementioned (1-6) as it is, or can be produced by purifying same and dissolving same in a solvent mainly containing water and approximately isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is, VEGF-positive cancer. The type of VEGF-positive cancer to be treated, diagnosed or detected by the present invention is not particularly limited as long as it expresses VEGF, and examples thereof include lung cancer, colorectal cancer, hepatocellular carcinoma, breast cancer, ovarian cancer, cervical cancer and malignant glioma. In addition, the cancer in which VEGF is expressed may be cancer at any stage, and may be either localized or metastatic, or primary or recurrent. As used herein, the "expression" refers to a state in which the VEGF gene in tumor tissue is significantly amplified as compared with non-tumor tissue, or the expression of VEGF protein is significantly enhanced as compared with non-tumor tissue, when measured by a known test method.

As used herein, the "effective amount" is an amount that can afford useful diagnostic or therapeutic effects in a subject of administration. The effective amount to be administered to a subject varies depending on the type of subject, body weight of the subject, dosage form of administration (tablet, injection, etc.) and route (oral administration, parenteral administration, etc.) of administration, severity of disease (e.g., cancer), and the like. Physicians and veterinarians can consider these factors and determine the appropriate effective amount.

The radiopharmaceutical of the present invention, when stored at room temperature, has radiochemical purity of not less than a given level when a time period of not less than 1 and not more than 5 times the half-life has passed, based on the half-life of the metal radionuclide constituting the radiopharmaceutical. When the above-mentioned metal radionuclide is a β-ray nuclide (e.g., Lu-177 or Y-90), the radiochemical purity of the conjugate when stored at room temperature for 7 days from the end of production is preferably not less than 90%, more preferably not less than 95%. When the metal radionuclide is an α-ray nuclide (e.g., Ac-225), the radiochemical purity of the conjugate when stored at room temperature for 14 days from the end of production is preferably not less than 90%, more preferably not less than 95%.

A specific embodiment of the radiopharmaceutical of the present invention contains a conjugate of a chelating agent to which metal radionuclide is chelated and an anti-VEGF antibody as an active ingredient, does not include a thiourea bond in the linking between the anti-VEGF antibody and the chelating agent (particularly linking via linker (L)), and, when stored at room temperature, has radiochemical purity of not less than a given level when a time period of not less than 1 and not more than 5 times the half-life has passed, based on the half-life of the metal radionuclide constituting the radiopharmaceutical. When the above-mentioned metal radionuclide is a β-ray nuclide (e.g., Lu-177 or Y-90), the radiochemical purity of the aforementioned conjugate when stored at room temperature for 7 days from the end of production is preferably not less than 90%, more preferably not less than 95%. When the metal radionuclide is an α-ray nuclide (e.g., Ac-225), the radiochemical purity of the conjugate when stored at room temperature for 14 days from the end of production is preferably not less than 90%, more preferably not less than 95%.

In the present specification, the "room temperature" preferably refers to "normal temperature" as defined in the Japanese Pharmacopoeia, and is specifically 15 to 25°C.

As used herein, the radiochemical purity refers to the percentage of the radioactivity (counts) of the peak corresponding to the conjugate relative to the total radioactivity (counts) detected when a sample is analyzed with a commercially available radiation detector. While high performance liquid chromatography or thin layer chromatography can be used to analyze radiochemical purity, thin layer chromatography is preferably used. More preferably, thin layer chromatography is used under the conditions described in the Examples below.

As mentioned above, the radiopharmaceutical of the present invention is preferably in the form of an aqueous solution. From the aspect of maintaining the radiochemical purity, it is more preferably in the form of a buffer solution. As the buffer solution, any buffer solution used in antibody drugs containing an anti-VEGF antibody or an ADC of an anti-VEGF antibody as an active ingredient can be used. As a nonlimiting example, a phosphate buffer solution, a succinate buffer solution, or a citrate buffer solution can be used. The phosphate buffer solution is composed of phosphoric acid and a salt thereof, and can be composed of, for example, phosphoric acid and a sodium salt thereof. The succinate buffer solution is composed of succinic acid and a salt thereof, and can be composed of, for example, succinic acid and a sodium salt thereof. The citrate buffer solution is composed of citric acid and a salt thereof, and can be composed of, for example, citric acid and a sodium salt thereof. The radiopharmaceutical of the present invention may contain any sugar such as trehalose, sucrose and the like, any amino acid such as histidine and the like, or a solubilizer such as polysorbate 20, polysorbate 80 and the like.

The radiopharmaceutical of the present invention can be used for radionuclide therapy of cancer by selecting metal radionuclides that have a therapeutic effect, specifically radionuclide that emits α rays or nuclide that emits β rays (preferably Ac-225, Y-90, Lu-177, more preferably Ac-225). In this radionuclide therapy, the radiopharmaceutical of the present invention is administered intravenously or orally to cause accumulation of the radioactive conjugate of the present invention in a lesion site such as a cancer primary lesion or metastatic lesion, and cancer cells at the lesion site are destroyed by the radiation emitted from the metal radionuclide. The amount of radioactivity to be administered and dose of the radiopharmaceutical of the present invention is appropriately determined by the efficacy of the active ingredient, the mode and route of administration, the stage of cancer progression, the body shape , body weight and age of the patient, and the type and amount of the therapeutic drug used in combination for other diseases.

In addition, by selecting a radionuclide that emits positron or a radionuclide that emits γ rays (preferably Ga-68, Zr-89, In-111) as the metal radionuclide, it can be used for cancer diagnosis or lesion detection. A radiopharmaceutical using radionuclide that emits positron can be preferably used for PET (Positron Emission Tomography)Scan, and a radiopharmaceutical using radionuclide that emits γ-rays can be preferably used for SPECT (Single Photon Emission Computed Tomography) Scan. This may also be used in combination with cancer diagnosis or lesion detection in the aforementioned radionuclide therapy of cancer. The diagnostic radiopharmaceutical for cancer of the present invention may be used for diagnosis before performing radionuclide therapy for cancer, or may be used for diagnosis after performing radionuclide therapy for cancer. Using the diagnostic radiopharmaceutical before conducting a radionuclide therapy for cancer, selection of treatment can be performed based on whether or not to perform radionuclide therapy for cancer using the radiopharmaceutical of the present invention provided with a radionuclide that emits α-rays. Using the diagnostic radiopharmaceutical after conducting a radionuclide therapy for cancer, moreover, whether or not radionuclide therapy for cancer using the radiopharmaceutical of the present invention is effective can be judged, and a treatment plan including an increase or a decrease of dose and the like can be optimized.

The following embodiments are also encompassed in the technical idea of the present invention.
[1] A conjugate of an antibody site specifically modified with an antibody-modification peptide and a chelating agent, wherein the aforementioned antibody is an anti-VEGF antibody, and a metal radionuclide is chelated to the aforementioned chelating agent.
[2] The conjugate of [1], wherein the aforementioned peptide has an amino acid sequence consisting of 13 to 17 amino acid residues and is represented by the following formula (i):

   (Xa) -Xaa₁- (Xb) -Xaa₂- (Xc) -Xaa₃- (Xd) ··· (i)

   (in the formula (i), Xa, Xb, Xc and Xd are continuous X in the number of a, continuous X in the number of b, continuous X in the number of c, and continuous X in the number of d, respectively,
   X is an amino acid residue having neither a thiol group nor a haloacetyl group in the side chain,
   a, b, c and d are each independently an integer of not less than one and not more than 5, and satisfy a+b+c+d≤14,
   Xaa₁ and Xaa₃ are each independently
   an amino acid residue derived from an amino acid having a thiol group in the side chain, or an amino acid residue derived from an amino acid having a haloacetyl group in the side chain, provided that either Xaa₁ or Xaa₃ is an amino acid residue derived from an amino acid having a thiol group,
   Xaa₁ and Xaa₃ are linked to form a ring structure, and
   Xaa₂ is a lysine residue, an arginine residue, a cysteine residue, an aspartic acid residue, a glutamic acid residue, 2-aminosuberic acid, or diaminopropionic acid, and modified with the crosslinking agent.
[3] The conjugate of [1] or [2], wherein the aforementioned metal radionuclide is Ga-68, Zr-89, Y-90, In-111, Lu-177, or Ac-225.
[4] The conjugate of any one of [1] to [3], wherein the aforementioned anti-VEGF antibody is bevacizumab.
[5] The conjugate of any one of [1] to [4], wherein the aforementioned peptide and the chelating agent are linked via a linker (L) and the linkage is a covalent bond that does not include a thiourea bond.
[6] The conjugate of any one of [1] to [5], wherein the aforementioned peptide and the chelating agent are linked via a linker (L), and the aforementioned linker (L) comprises the formula (10a), the formula (10b), or the formula (10c). 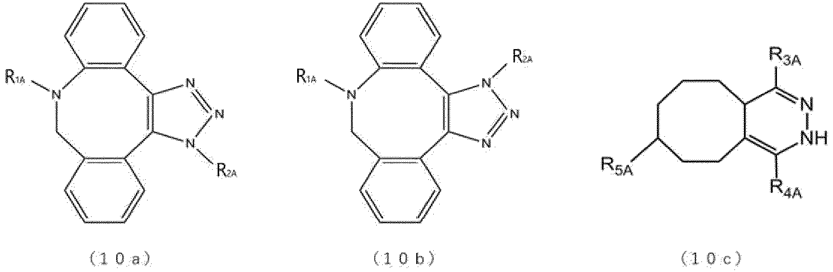 (in the formula (10a) and the formula (10b), R_{1A} is a linkage site with the aforementioned chelating agent, R_{2A} is a linkage site with the aforementioned peptide, in the formula (10c), one of R_{3A} and R_{4A} is a hydrogen atom, a methyl group, a phenyl group, or a pyridyl group, and the other is a linkage site with the aforementioned chelating agent, and R_{5A} is a linkage site with the aforementioned peptide).
[7] The conjugate of [6], comprising a polyethylene glycol group between the linkage site with the aforementioned peptide and the aforementioned peptide.
[8] The conjugate of any one of [1] to [6], wherein the aforementioned chelating agent is DOTAGA (α-(2-carboxyethyl)-1,4,7,10-tetraazacyclododecane-1,4,7,10-tetraacetic acid).
[9] The conjugate of any one of [1] to [8], which is conjugated by a click reaction between an anti-VEGF antibody site-specifically modified with the aforementioned peptide having an azide group introduced into the N-terminus with a radioactive metal complex of DOTAGA-DBCO represented by the following formula: 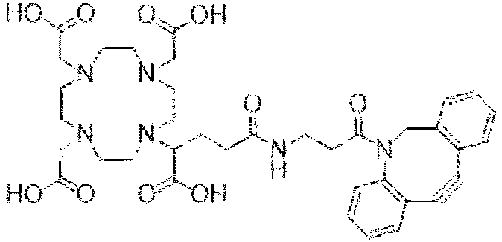 DOTAGA-DBCO
[10] A radiopharmaceutical comprising the conjugate of any one of [1] to [9] as an active ingredient.
[11] The radiopharmaceutical of [10], which is used for cancer radionuclide therapy.
[12] The radiopharmaceutical of [10], which is used for cancer diagnosis.
[13] The radiopharmaceutical of [12], which is used in combination in cancer radionuclide therapy using the radiopharmaceutical of [11].
[14] A radiopharmaceutical comprising a conjugate of a chelating agent to which a metal radionuclide is chelated and an anti-VEGF antibody as an active ingredient, not comprising a thiourea bond in a linkage between the anti-VEGF antibody and the chelating agent, and satisfying the following condition (1) or (2) :
   (1) the aforementioned metal radionuclide is ¹⁷⁷Lu or ⁹⁰Y, and the aforementioned conjugate has a radiochemical purity of not less than 90% when stored at room temperature for 7 days
   (2) the aforementioned metal radionuclide is ²²⁵Ac, and the aforementioned conjugate has a radiochemical purity of not less than 90% when stored at room temperature for 14 days.
[15] A radiopharmaceutical comprising a conjugate of a chelating agent to which a metal radionuclide is chelated and an anti-VEGF antibody as an active ingredient, not comprising a thiourea bond in a linkage between the anti-VEGF antibody and the chelating agent, and having a radiochemical purity of the aforementioned conjugate of not less than 90% when a time period of not less than 1 and not more than 5 times the aforementioned half-life has passed, based on the half-life of the aforementioned metal radionuclide.

### [Example]

The present invention is explained in detail in the following by referring to Examples. The present invention is not limited to such Examples.

### [Example 1] Production of site-specifically ²²⁵Ac-labeled conjugate of DOTAGA and bevacizumab (²²⁵Ac-CCAP-bevacizumab1) (1) Antibody modification step

A peptide containing 17 amino acid residues represented by the following formula (P3) was obtained by the method described in WO 2017/217347. The amino acid sequence of this peptide was the same as the sequence in which Xaa₂ of SEQ ID NO: (2) was a lysine residue, and the side chain terminal amino group of the lysine residue was modified with the structure shown by R₁. In addition, two cysteine residues form a disulfide bond with each other, and to the N-terminal of the peptide was added ethyl azide as an atomic group containing an azide group, which is the second atomic group, via a linker (L₁) structure having diglycolic acid and eight PEGs.

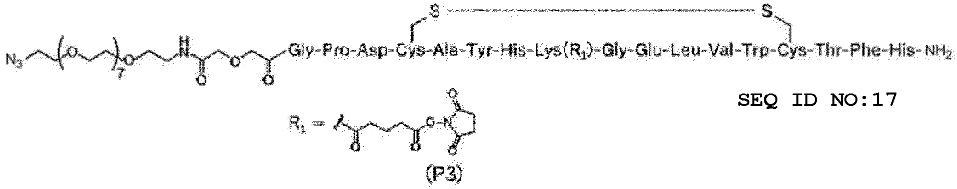

In the formula (P3), Gly is glycine, Pro is proline, Asp is aspartic acid, Cys is cysteine, Ala is alanine, Tyr is tyrosine, Lys is lysine, His is histidine, Glu is glutamic acid, Leu is leucine, Val is valine, Trp is tryptophan, Thr is threonine, and Phe is phenylalanine.

A mixture of the aforementioned peptide and bevacizumab (manufactured by Roche) in 0.02 mol/L acetic acid·sodium acetate buffer (pH 6.0) was reacted at room temperature for 60 min to give a solution containing a peptide-modified antibody. An Fc region of the peptide-modified antibody is site-specifically modified by the above-mentioned peptide.

Then, the solution was passed through an IgG-BP column to obtain a first antibody composition containing unlabeled antibodies and monovalent antibodies in relatively large amounts. The concentration was adjusted with a preservation buffer (mixed solution of 60 g/L trehalose and 5.8 g/L sodium dihydrogen phosphate monohydrate, anhydrous sodium monohydrogen phosphate, 0.4 g/L polysorbate 20) such that the concentration of the monovalent antibody contained in the recovered fraction was 14.6 mg/mL. The solution containing the obtained first antibody composition was subjected to the labeling step described below.

### (2. Complex formation step)

DOTAGA-DBCO represented by the following formula was produced based on the method described in Bernhard et al. DOTAGA-Anhydride: A Valuable Building Block for the Preparation of DOTA-Like Chelating Agents. Chem. Eur. J. 2012, 18, 7834-7841. This chelating agent was dispersed in 0.1 mol/L sodium acetate buffer (pH 5.0) as a solvent to give a dispersion containing 0.3 mmol/L chelating agent. A reaction mixture of the dispersion (0.028 mL) and ²²⁵Ac ion-containing solution (0.1 mol/L aqueous hydrochloric acid solution, radioactivity concentration 237.6 MBq/mL, prepared from one produced by State Atomic Energy Corporation Rosatom, liquid amount 0.0126 mL) about 2.99 MBq (Calculated value corrected for attenuation from the amount of radioactivity at the time of the inspection) as a radioactive metal source was reacted under heating conditions to give a ²²⁵Ac complex solution. The molar ratio of the chelating agent and the radioactive metal ion was chelating agent:²²⁵Ac ion = about 1350:1, and the heating temperature of the reaction mixture was set to 70°C, and the heating time was set to 30 min.

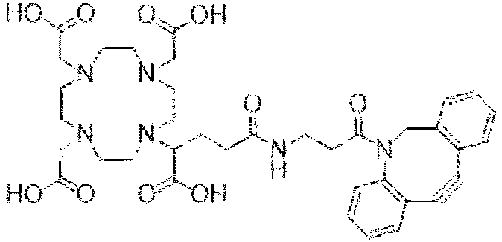

### DOTAGA-DBCO

The radiochemical purity (RCP, %) of the obtained ²²⁵Ac complex was measured by the following method. That is, a part of the ²²⁵Ac complex solution was developed by thin layer chromatography (Agilent, model number: SGI0001, developing solvent: acetonitrile/water mixture (volume ratio 1:1)), and then measured by a radio γ-TLC analyzer (Raytest, Model GITA Star). The percentage of the peak radioactivity (counts) detected near the point of origin relative to the total detected radioactivity (counts) was taken as the RCP of the ²²⁵Ac complex. As a result, the RCP of the ²²⁵Ac complex was 79%. The obtained ²²⁵Ac complex solution was used as it was in the labeling step.

### (3. Labeling step)

A solution containing the peptide-modified antibody (monovalent antibody) obtained in the above-mentioned step (1) was added to the solution of the unpurified ²²⁵Ac complex obtained through the aforementioned step (2), and a click reaction was performed at 37°C for 120 min to obtain ²²⁵Ac-CCAP-bevacizumab1. The amount of the ²²⁵Ac complex and the amount of the peptide-modified antibody (monovalent antibody) were 8.7 nmol and 10.2 nmol, respectively, and the molar ratio of the DBCO group to the azide group was about 1:1.2, respectively. The reaction rate (%) of the unpurified ²²⁵Ac-CCAP-bevacizumab1 was 72%. As used herein, the reaction rate (%) means the RCP of ²²⁵Ac-CCAP-bevacizumab1 relative to the labeling rate (%) in the complex formation step, and the labeling rate (%) means the ratio (%) of the radioactivity of the ²²⁵Ac complex to the input radioactivity.

Furthermore, the solution of ²²⁵Ac-CCAP-bevacizumab1 obtained by reacting at 37°C for 2 hr was purified using an ultrafiltration filter (Merck, model number: UFC505096). After purification, the radiochemical yield (RCY, %) of ²²⁵Ac-CCAP-bevacizumab1 was 70%, and RCP was 97%.

The reaction rate, RCP, and RCY of ²²⁵Ac-CCAP-bevacizumab1 were measured as follows. That is, thin layer chromatography (Agilent, model number: SGI0001, developing solvent was a mixture of acetonitrile:0.1 mmol/L EDTA solution (volume ratio 1:1)) was measured with a radio-γ-TLC analyzer (Raytest, MODEL GITA Star). The percentage of the peak radioactivity (counts) detected near the point of origin relative to the total radioactivity (counts) detected was taken as RCP (%), which was divided by the labeling rate (%) to calculate the reaction rate (%). Furthermore, the percentage of the radioactivity recovered after ultrafiltration purification (the amount of radioactivity calculated from the counts measured with a γ-ray spectrometer (Ge semiconductor detector: GMX10P4-70 (manufactured by ORTEC), multichannel analyzer: M7-000 (manufactured by Seiko E.G. & G), data processing: Spectrum Navigator: DS-P300 (manufactured by Seiko E.G. & G) and Gamma Studio: DS-P600 (manufactured by Seiko E.G. & G)) relative to the total radioactivity added at the start of the labeling step (the amount of radioactivity calculated from the counts measured with a γ-ray spectrometer in the same manner as above) was defined as RCY (%).

### [Example 2] Production of site-specifically ²²⁵Ac-labeled conjugate of DOTA and bevacizumab (²²⁵Ac-CCAP-bevacizumab2)

²²⁵Ac-CCAP-bevacizumab2 was obtained by an operation according to Example 1, except that DOTAGA-DBCO was replaced with DOTA-Bn-DBCO below. The reaction rate was 50%, RCY was 15%, and RCP was 62%. 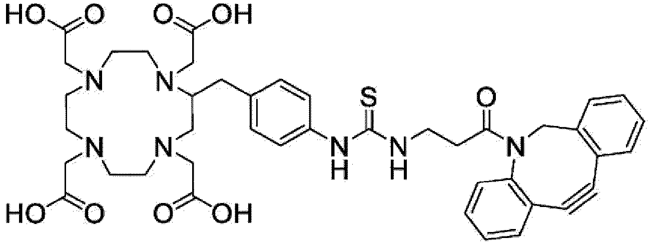 DOTA-Bn-DBCO

### [Example 3] Preparation step

A portion of ²²⁵Ac-CCAP-bevacizumab1 produced according to the description of Example 1 was taken out into a 5 mL Eppendorf tube (LoBind, manufactured by Eppendorf) and diluted with formulation buffer (mixed solution of 60 g/L trehalose, 5.8 g/L sodium dihydrogen phosphate monohydrate, anhydrous sodium monohydrogen phosphate, and 0.4 g/L polysorbate 20).

### [Evaluation 1] In vitro efficacy evaluation

The in vitro efficacy of the radioactive conjugate of the present invention was evaluated using the following lung cancer-derived cell lines.
· A549 (human lung glandular cancer; source: ECACC (The European Collection of Authenticated Cell Cultures); F-12K medium)
· NCI-H358 (human lung glandular cancer; source: ATCC (American Type Culture Collection); RPMI 1640 medium)
· NCI-H520 (human lung squamous cell carcinoma; source: ATCC; RPMI1640 medium)
· NCI-H460 (human non-small cell lung cancer; source: ATCC; RPMI1640 medium)

The aforementioned cells were cultured in appropriate media, and ²²⁵Ac-CCAP-bevacizumab1 prepared according to the description in Example 3 was diluted with each medium to 0, 0.001, 0.01, 0.03, 0.1, 0.3, 1, 3, 10, and 30 kBq/mL (0.00229, 0.0229, 0.0687, 0.229, 0.687, 2.29, 6.87, 22.9, and 68.7 ug/mL as bevacizumab) and added to the cells. Unlabeled bevacizumab was also added to the cells so that the antibody concentration was the same as that of the samples with each radioactivity concentration, and the cells were cultured. After 120 hr from the addition of the sample, CellTiter-Glo (registered trademark) 2.0 Cell Viability Assay (manufactured by Promega) was added to the medium, and chemiluminescence was detected using a microplate reader (SpectraMax i3x, manufactured by Molecular Devices) to calculate the number of viable cells. The cytotoxic effect was evaluated by calculating the ratio of the calculated number of viable cells to the number of viable cells in the case of the condition in which the antibody was not added.

The results are shown in Fig. 1. The vertical axis shows the relative value when the number of viable cells without the addition of antibody is taken as 100%, and the horizontal axis shows the concentration of the added antibody. Compared to the original antibody bevacizumab (unlabeled) (O in the Figure), ²²⁵Ac-CCAP-bevacizumab1 (● in the Figure) was confirmed to have a cytotoxic effect even with a small amount of antibody administered. In addition, the cytotoxic effect dependent on the added radioactivity was confirmed.

### [Evaluation 2] In vivo efficacy evaluation

A subcutaneous A549 tumor-bearing model was created using mice and the in vivo efficacy of the radioactive conjugate of the present invention was evaluated.

A549 cells, a VEGF-positive cell line derived from human lung adenocarcinoma purchased from ATCC, were suspended in F-12K medium and 5×10⁶ cells were subcutaneously transplanted into the flank of 5-week-old female BALB/c nu/nu mice (manufactured by Jackson Laboratory Japan, Inc.) to create tumor-bearing mice. Tumors were allowed to grow until they reached a volume of 100 - 300 mm³, and mice with a shape suitable for tumor diameter measurement were randomly divided into groups. The tumor volume and body weight of each mouse at that time are shown in Table 1. Tumor volume was calculated according to the following formula. tumor volume (mm3) = (tumor long diameter × (tumor short diameter)2) × 1/2

**[Table 1]**

| | tumor volume mean±standard deviation (mm³) | body weight mean±standard deviation (g) |
|---|---|---|
| ²²⁵Ac-CCAP-bevacizumab1 group | 136.3±45.3 | 23.0±1.3 |
| antibody control group | 130.4±37.9 | 23.4±1.5 |
| radioactivity control group | 130.7±39.3 | 23.5±1.0 |
| Vehicle group | 127.2±37.2 | 22.5±1.0 |

²²⁵Ac-CCAP-bevacizumab1 was administered into the tail vein at a dose of 10 kBq/mouse (60 ug/mouse as bevacizumab).

Antibody control group, radioactivity control group, and vehicle group were each set as control groups.

The antibody control group was administered with bevacizumab (unlabeled) in the same antibody amount as ²²⁵Ac-CCAP-bevacizumab1.

The radioactivity control group was administered with ²²⁵Ac-CCAP-human IgG antibody at a dose of 10 kBq/mouse, which was produced in the same manner as in Example 1, except that bevacizumab was replaced with human IgG antibody (InVivoPlus human IgG1 isotype control, manufactured by Bio X cell).

The vehicle group was administered only with the formulation buffer used in Example 3.

Each group was administered at a dose of 100 µL/mouse.

Each group consisted of 5 or 6 mice, and general conditions were observed, and the body weight and tumor volume were measured over time up to 42 days after administration. The change in tumor volume over time is shown in the graph in Fig. 2. The vertical axis of Fig. 2 represents relative values when the tumor volume at the time of administration of each drug is set to 1, and the horizontal axis of Fig. 2 represents the number of days since administration of each drug. The graph shows the mean tumor volume for each group ± standard deviation, with "*" indicating the time point at which a significant difference (p<0.01) was observed compared to the vehicle group, and "†" indicating the time point at which a significant difference (p<0.05) was observed compared to the radioactive control group.

The group administered with ²²⁵Ac-CCAP-bevacizumab1 showed a significant difference in antitumor effect compared to the radioactive control group and the vehicle group at the 42nd day after administration (P<0.05 and P<0.01). To test for significant difference, a Tukey test was performed using statistical analysis software Stat Preclinica (manufactured by Takumi Information Technology Co., Ltd.). On the other hand, ²²⁵Ac-CCAP-bevacizumab1 showed no significant difference in antitumor effect compared to the antibody control group, but showed a tendency toward a stronger antitumor effect. In addition, there was no remarkable change in the general condition of each group, and no signs of toxicity such as remarkable weight loss and the like were observed.

### [Example 4] Production of site-specifically ⁸⁹Zr-labeled conjugate of DOTAGA and bevacizumab (⁸⁹Zr-CCAP-bevacizumab1)

### (1. Complex formation step)

DOTAGA-DBCO was dispersed in 0.1 mol/L sodium acetate buffer (pH 5.0) as a solvent to give a dispersion containing 0.3 mmol/L chelating agent. A reaction solution obtained by mixing 0.1086 mL of this dispersion with 0.0815 mL of a 0.156 mol/L sodium acetate solution containing 0.075 mol/L gentisic acid and 317 MBq of a solution containing ⁸⁹Zr ions (0.1 mol/L hydrochloric acid aqueous solution, radioactivity concentration 5.76 GBq/mL, prepared from one manufactured by Nippon Medi-Physics Co., Ltd., liquid volume 0.055 mL) as a radioactive metal source was reacted under heating conditions to obtain a ⁸⁹Zr complex solution. The molar ratio of the chelating agent and the radioactive metal ion was chelating agent:⁸⁹Zr ion = about 152:1, and the heating temperature of the reaction mixture was set to 70°C, and the heating time was set to 60 min. The obtained ⁸⁹Zr complex solution was used as it was in the labeling step. The RCP of the obtained ⁸⁹Zr complex was measured by the following method. That is, a part of the ⁸⁹Zr complex solution was developed by thin layer chromatography (manufactured by Agilent, model number: SGI0001, developing solvent was acetonitrile:water mixture (volume ratio 1:1)), and then measured by a radio γ-TLC analyzer (manufactured by Raytest, Model GITA Star PS). The percentage of the radioactivity (counts) of the peak detected near the point of origin to the total radioactivity (counts) detected was taken as the RCP (%) of the ⁸⁹Zr complex. As a result, the RCP of the ⁸⁹Zr complex was 74%. The obtained ⁸⁹Zr complex solution was used as it was in the labeling step.

### (2. Labeling step)

A solution containing the peptide-modified antibody (monovalent antibody) obtained in the same manner as in Example 1 was added to the solution of the unpurified ⁸⁹Zr complex obtained through the aforementioned step (1) were respectively added unpurified to 0.1 mol/L arginine-containing 0.05 mmol/L histidine buffer (pH 6.1), and a click reaction was performed at 37°C for 90 min to obtain ⁸⁹Zr-CCAP-bevacizumab1. The amount of the ⁸⁹Zr complex and the amount of the peptide-modified antibody (monovalent antibody) were 32.6 nmol and 24.6 nmol, respectively, and the molar ratio of the DBCO group to the azide group was about 1:0.75, respectively. The reaction rate (%) of the unpurified ⁸⁹Zr-CCAP-bevacizumab1 was 74%. As used herein, the reaction rate (%) means the RCP of ⁸⁹Zr complex relative to the labeling rate (%) in the complex formation step, and the labeling rate (%) means the ratio (%) of the radioactivity of the ⁸⁹Zr complex to the input radioactivity.

Furthermore, the solution of ⁸⁹Zr-CCAP-bevacizumab1 obtained by reacting at 37°C for 2 hr was purified using an ultrafiltration filter (Merck, model number: UFC505096). After purification, RCP of ⁸⁹Zr-CCAP-bevacizumab1 was 96%, and RCY was 62%. RCP and RCY of ⁸⁹Zr-CCAP-bevacizumab1 were measured as in Example 1.

### [Example 5] Preparation step

1.0 mL of each of the radioactive conjugates produced according to the description in Example 4 was dispensed into a 5 mL Eppendorf tube (LoBind, manufactured by Eppendorf) and diluted with 3.64 mL of formulation buffer (mixed solution of 42 g/L trehalose hydrate, 0.47 g/L L-histidine hydrochloride hydrate, 0.30 g/L L-histidine, and 85 mg/L polysorbate 20).

### [Evaluation 3] In vivo tumor accumulation

In addition to the cells used in Evaluation 2, the following lung cancer cell line was used to create tumor-bearing mice.
- NCI-H209 (human small cell lung cancer; source: ATCC; RPMI 1640 medium)

A cell suspension of each cell line was prepared, and 5×10⁶ cells were subcutaneously transplanted into the left front leg of a 5-week-old female BALB/c nu/nu (Charles River Laboratories Japan, Inc.) to create a tumor-bearing mouse. The tumor was allowed to grow until the tumor volume reached about 100 - 300 mm³, and ⁸⁹Zr-CCAP-bevacizumab1 was administered into the tail vein at a dose of 6 MBq/mouse (n=3 each). 72 hours after administration, imaging was performed using a small animal PET imaging device (PET/CT Si78, manufactured by Bruker). The PET imaging conditions were Acquisition time of 600 sec and Energy window of 30% (357.7-664.3 keV). Data correction included scatter correction, random correction, attenuation correction, partial volume, and attenuation, and PET image reconstruction was performed using the maximum likelihood-expectation maximization (MLEM) method (Iterations: 12). Tumor volume was calculated using the following formula: Tumor volume (mm3) = (tumor long diameter × (tumor short diameter)2) × 1/2

A representative example of the results of PET imaging is shown in Fig. 3. In Fig. 3, the arrow indicates the tumor site.

Radioactivity accumulated at a higher concentration in the tumor as compared with other organs, and VEGF-positive tumor could be visualized.

### [Evaluation 4] Stability evaluation

Each radioactive conjugate (²²⁵Ac-CCAP-bevacizumab1 and ²²⁵Ac-CCAP-bevacizumab2) produced according to the descriptions in Example 1 and Example 2 and prepared according to the description in Example 3 was stored at room temperature (24.8-24.9°C) for 2 weeks, and RCP and the proportion of aggregates were evaluated at each time point (0th day, 1st day, 7th day, and 14th day). When the radioactive metal nuclide is ²²⁵Ac, the period of 14 days after completion of the production corresponds to about 1.5 half-life.

### [Evaluation 4-1] Radiochemical purity

RCP was analyzed by thin layer chromatography (TLC). The TLC conditions were similar to those used when examining the reaction rate in Example 1.

The results are shown in Table 2.

**[Table 2]**

| | RCP (%) | | | |
|---|---|---|---|---|
| | 0th day | 1st day | 7th day | 14th day |
| ²²⁵Ac-CCAP-bevacizumab1 | 100.0 | 99.8 | 98.3 | 97.8 |
| ²²⁵Ac-CCAP-bevacizumab2 | 96.8 | 91.9 | 73.8 | 75.5 |

For ²²⁵Ac-CCAP-bevacizumab1, the RCP was maintained at 98% or more when stored at room temperature for 7 days after the completion of the production, and no significant decrease in RCP was observed even when stored for 14 days.

For ²²⁵Ac-CCAP-bevacizumab2, the RCP was below 80% when stored at room temperature for 7 days or more after the completion of the production.

The proportion of the aggregates was confirmed by size-exclusion chromatography (SEC). Using Model 2695 Separation Module or e2695 Separation Module, manufactured by Water, as a liquid chromatography device, and Model 2489 UV/Vis Detector manufactured by Waters as a UV detector, analysis was performed under the following conditions. The proportion of each component when stored for 14 days after completion of the production is shown in Table 3. When stored for 14 days after completion of the production, The proportion of the aggregates was of the same level between ²²⁵Ac-CCAP-bevacizumab1 and ²²⁵Ac-CCAP-bevacizumab2.

### [HPLC conditions]

column: TOSOH TSK gel guard column SWXL (6 mm×4 cm),TOSOH TSK gel G3000SWXL (5 µm, 7.8×30 cm)×2 (tandem)
column temperature: constant temperature around 25°C
mobile phase: 0.2 mol/L arginine hydrochloride-containing 0.1 mol/L sodium dihydrogen phosphate buffer (pH 6.8)
flow: 1.0 mL per minute
area measurement range: 30 min
detection wavelength: 280 nm

**[Table 3]**

| | | proportion of main peak (%) | proportion of aggregate peak (%) |
|---|---|---|---|
| ²²⁵Ac-CCAP-bevacizumab1 | 0th day | 98.6 | 0.3 |
| | 1st day | 99.4 | 0.2 |
| | 7th day | 98.5 | 1.1 |
| | 14th day | 95.9 | 3.2 |
| ²²⁵Ac-CCAP-bevacizumab2 | 0th day | 98.0 | 0.8 |
| | 1st day | 99.1 | 0.0 |
| | 7th day | 94.7 | 1.4 |
| | 14th day | 92.1 | 1.5 |

This application is based on a patent application No. 2022-021663 filed in Japan (filing date: February 15, 2022), the contents of which are incorporated in full herein.

## Claims

1. A conjugate of an antibody site-specifically modified with an antibody-modification peptide and a chelating agent, wherein
the antibody is an anti-VEGF antibody, and
a metal radionuclide is chelated to the chelating agent.

2. The conjugate according to claim 1, wherein the peptide has an amino acid sequence consisting of 13 to 17 amino acid residues and is represented by the following formula (i):
(Xa) -Xaa₁- (Xb) -Xaa₂- (Xc) -Xaa₃- (Xd) ··· (i)
(in the formula (i), Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa₁ and Xaa₃ are each independently
an amino acid residue derived from an amino acid having a thiol group in the side chain, or an amino acid residue derived from an amino acid having a haloacetyl group in the side chain,
provided that either Xaa₁ or Xaa₃ is an amino acid residue derived from an amino acid having a thiol group,
Xaa₁ and Xaa₃ are linked to form a ring structure, and
Xaa₂ is a lysine residue, an arginine residue, a cysteine residue, an aspartic acid residue, a glutamic acid residue, 2-aminosuberic acid, or diaminopropionic acid, and modified with the crosslinking agent.

3. The conjugate according to claim 1 or 2, wherein the metal radionuclide is Ga-68, Zr-89, Y-90, In-111, Lu-177, or Ac-225.

4. The conjugate according to claim 1, wherein the anti-VEGF antibody is bevacizumab.

5. The conjugate according to claim 1, wherein the peptide and the chelating agent are linked via a linker (L) and the linkage is a covalent bond that does not include a thiourea bond.

6. The conjugate according to claim 1, wherein the peptide and the chelating agent are linked via a linker (L), and the linker (L) comprises the formula (10a), the formula (10b), or the formula (10c). 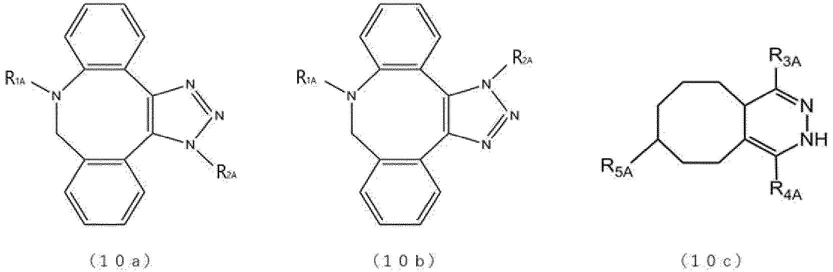 (in the formula (10a) and the formula (10b), R_{1A} is a linkage site with the chelating agent, R_{2A} is a linkage site with the peptide, in the formula (10c), one of R_{3A} and R_{4A} is a hydrogen atom, a methyl group, a phenyl group, or a pyridyl group, and the other is a linkage site with the chelating agent, and R_{5A} is a linkage site with the peptide).

7. The conjugate according to claim 6, comprising a polyethylene glycol group between the linkage site with the peptide and the peptide.

8. The conjugate according to claim 1, wherein the chelating agent is DOTAGA (α-(2-carboxyethyl)-1,4,7,10-tetraazacyclododecane-1,4,7,10-tetraacetic acid).

9. The conjugate according to claim 1, which is conjugated by a click reaction between an anti-VEGF antibody site-specifically modified with the peptide having an azide group introduced into the N-terminus with a radioactive metal complex of DOTAGA-DBCO represented by the following formula: 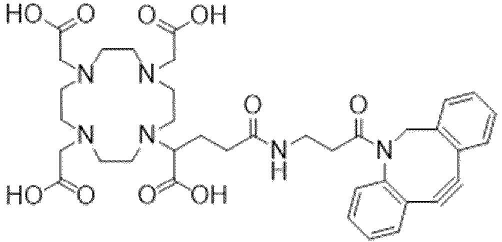 DOTAGA DBCO

10. A radiopharmaceutical comprising the conjugate according to any one of claims 1 to 9 as an active ingredient.

11. The radiopharmaceutical according to claim 10, which is used for cancer radionuclide therapy.

12. The radiopharmaceutical according to claim 10, which is used for cancer diagnosis.

13. The radiopharmaceutical according to claim 12, which is used in combination in cancer radionuclide therapy using the radiopharmaceutical according to claim 11.
